# EUROPEAN PATENT APPLICATION

(11) **EP 0 775 477 A1**
(43) Date of publication of application: **28.05.1997**
(21) Application number: 95308409.2
(22) Date of filing: 23.11.1995
(51) Int. Cl.: A61F 13/02

(54) **Self-retaining bandage**

(71) Applicant: Ueda, Shiro, Taipei (TW)
(72) Inventor: Ueda, Shiro, Taipei (TW)
(74) Representative: Jennings, Nigel Robin

(57) **Abstract**

A self-retaining bandage having a reticular strap (10) of elastomeric material with a plurality of concavities (11) and convexities (12) evenly disposed on the upper and lower surfaces of the reticular strap. The upper and lower surfaces are also coated with silica gel whereby a wound is bandaged by winding the bandage around it and superimposing two portions of the bandage which are then connected together by means of the opposed concavities (11) and convexities (12) on the superimposed portions interlocking with one another with the coating of silica gel providing adhesion therebetween.

## Description

The present invention relates to a self-retaining bandage, more particularly to a bandage having a coating of silica gel on an upper surface and a lower surface thereof for concavities and convexities configurations evenly disposed thereon respectively to attach to each other so as to bind up a wound.

In everyday life, it is hard for a person to avoid suffering accidents. When one is injured, a wound needs binding up with a gauze. Prior art gauze can prevent the wound from being infected, however, the gauze needs cutting after binding up the wound and in general, this requires the aid of another person. Second, prior art gauze is abandoned after being used so that the cost of the medical treatment increases.

It is therefore an object of the present invention to provide a kind of self-retaining bandage which can be simply used without being cut.

It is another object of the invention to provide a kind of bandage which can be re-used.

A further object of the invention is to provide a kind of bandage which is easy to put in order when unused.

Briefly stated, in accordance with one aspect of the invention the foregoing objects are achieved by providing a kind of self-retaining bandage comprising a reticular strap made of elastomer. On the strap is a coating of silica gel for adhesion. A plurality of part-spherical concavities and a corresponding plurality of part-convexities are evenly disposed on an upper surface and a lower surface of the reticular strap respectively to attach to each other with the coating of silica gel providing adhesion therebetween when the bandage is used to bind up a wound.
In the drawings:
FIG. 1 is a plane figure of the bandage in accordance with the present invention;
FIG. 2 is a schematic view of the preferred embodiment when the bandage is used for binding up a wound of the invention; and
FIG. 3 is a cross-sectional view of the bandage showing the respective attachment of the concavities and the convexities.

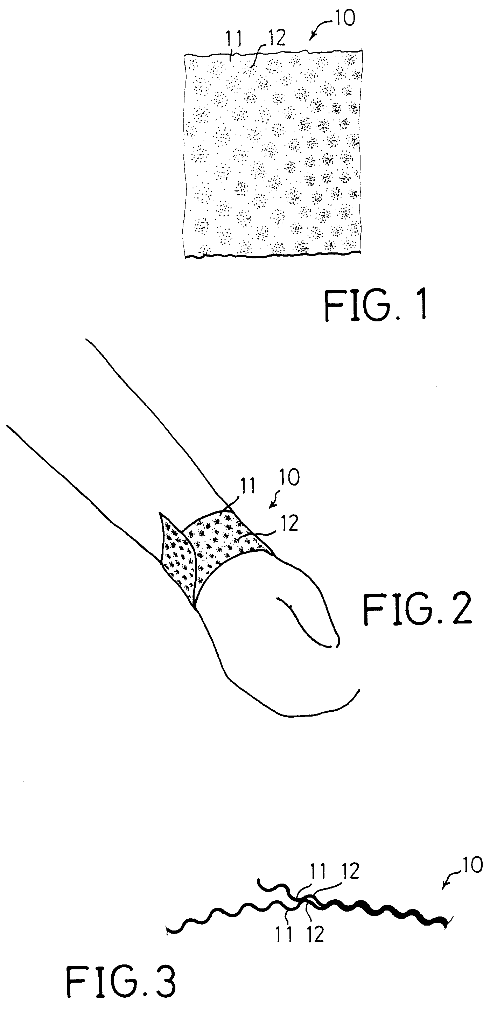

FIG. 1 depicts a kind of self-retaining bandage which comprises a reticular strap 10 made of elastomer, a plurality of part-spherical concavities 11 and a corresponding plurality of part-spherical convexities 12 which are sized and configured to cooperate with the part-spherical concavities are evenly disposed on an upper surface and a lower surface of the reticular strap 10 wherein the upper and lower surfaces are coated with a silica gel (not numbered).

Referring to FIG. 2 and FIG. 3, there shows a preferred embodiment of the bandage. When a wound (not numbered) is bound up with the bandage, the concavities 11 and the convexities 12 on the lower surf ace around the arm respectively attach to the convexities 12 and the concavities 11 on the upper surface with the coating of silica gel providing adhesion therebetween.

In addition, the bandage in accordance with the present invention can be re-used after cleaning and sterilization.

The present disclosure includes that contained in the appended claims as well as that of the foregoing description. Although this invention has been described in its preferred forms with a certain degree of particularity, it is understood that the present disclosure of the preferred form has been made only by way of example and numerous changes in the details of construction and combination and arrangement of parts may be resorted to without departing from the spirit and scope of the invention.

## Claims

1. A self-retaining bandage comprising:
a reticular strap made of a resilient material;
a plurality of concavities and and a corresponding plurality of convexities sized and configured to cooperate with each other and evenly disposed on an upper surface and a lower surface of the reticular strap; and
a coating of silica gel applied to the upper surface and lower surface of the strap whereby one part of the strap securely adheres to another part of the strap when said one part is brought to be retained to said another part with mutually facing concavities and convexities respectively on said parts aligning with one another.

2. The bandage of claim 1, wherein the plurality of concavities and the plurality of convexities are each part-spherical of a same size.

3. The bandage of claim 1, wherein the strap is made of elastomer.
